(19) Europäisches Patentamt
European Patent Office
Office européen des brevets

(11) **EP 4 177 907 A1**

(12) **EUROPEAN PATENT APPLICATION**

(43) Date of publication:
**10.05.2023 Bulletin 2023/19**

(21) Application number: **21306550.1**

(22) Date of filing: **04.11.2021**

(51) International Patent Classification (IPC):
***G16H 50/30*** *(2018.01)*

(52) Cooperative Patent Classification (CPC):
**G16H 50/30**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Essilor International**
**94220 Charenton-Le-Pont (FR)**

(72) Inventors:
• **MARIN, Gildas**
**75012 PARIS (FR)**
• **DROBE, Bjorn**
**479251 SINGAPORE (SG)**

(74) Representative: **Jacobacci Coralis Harle**
**32, rue de l'Arcade**
**75008 Paris (FR)**

(54) **A METHOD AND SYSTEM FOR DETERMINING A RISK OF AN ONSET OR PROGRESSION OF MYOPIA**

(57) The disclosure concerns methods for determining a risk of an onset or progression of myopia over a timeframe. The method includes determining a value of at least one parameter associated with vision condition of a subject, said at least one parameter comprising a sensitivity parameter of said subject, said sensitivity parameter being relative to the sensitivity of said subject to a variation of at least one dioptric optical feature of at least one ophthalmic lens placed in front of at least one eye of said subject. The method also includes determining the subject's risk of the onset or progression of myopia over the timeframe, based on the determined value of the at least one parameter. A system for determining a risk of an onset or progression of myopia over a timeframe is also disclosed.

<u>100</u>

Determine a value of at least one parameter associated with subject's vision condition — 110

Determine subject's risk of onset or progression of myopia over a timeframe — 120

FIG. 1

EP 4 177 907 A1

**Description**

**TECHNICAL FIELD**

Various aspects of this disclosure relate to a method for determining a risk of an onset or progression of myopia over a timeframe. Various aspects of this disclosure further relate to a system for determining a risk of an onset or progression of myopia over a timeframe.

**BACKGROUND**

Myopia, a form of ametropia, is a condition of the human eye where the light that comes in does not directly focus on the retina but in front of it, causing an image that one sees to be out of focus when looking at a distant object, but in focus when looking at a close object. Low myopia (e.g. > -5 D) and high myopia are both associated with increasing risks of ocular pathologies, severe vision defects and in extreme cases, blindness. Thus, myopia onset and control of myopia progression has become a serious burden both in the clinical and research domains of eye care.

Myopia is a complex condition which involves many factors, and evaluating the progression speed of myopia requires continuous follow-up over a long duration. In addition, predicting the onset of myopia typically is highly complicated. Several models allow the prediction of myopia onset and progression, but with poor accuracy and reliability. In particular, these models fail to explain a large part of the variance observed in pre-myopic and myopic populations, suggesting that an important factor is missing in these predictive models.

Thus, it is desired to seek more accurate and reliable means to determine a risk of myopia onset or progression over a timeframe, in pre-myopic and myopic populations.

**SUMMARY**

It is an object of the invention to provide methods and a system to accurately and reliably determine a subject's risk of an onset or progression of myopia over a timeframe. To this end, methods and a system are provided, in which determination of a subject's risk of an onset or progression of myopia over a timeframe may be based on at least, a determined value of a sensitivity parameter of a subject.

A first aspect of the disclosure concerns a method for determining a risk of an onset or progression of myopia over a timeframe. The method includes determining a value of at least one parameter associated with vision condition of a subject, said at least one parameter comprising a sensitivity parameter of said subject, said sensitivity parameter being relative to the sensitivity of said subject to a variation of at least one dioptric optical feature of at least one ophthalmic lens placed in front of at least one eye of said subject. The method also includes determining the subject's risk of the onset or progression of myopia over the timeframe, based on the determined value of the at least one parameter.

According to various embodiments, the at least one parameter associated with vision condition of the subject may further include: a dioptric optical parameter, a parameter relating to the subject's lifestyle, activity or behavior, a parameter relating to the subject's genetic history, an optical biometric parameter, and/or a parameter relating to personal information about the subject. In some embodiments, the parameters associated with vision condition of the subject may include all the aforementioned parameters and the sensitivity parameter of said subject.

According to various embodiments, determining the subject's risk of the onset or progression of myopia over the timeframe, based on the determined value of the at least one parameter may include assigning to each of the at least one parameter, a respective risk category selected from a plurality of predetermined risk categories, based on the determined value of the respective parameter, and may further include determining the subject's risk of the onset or progression of myopia over the timeframe, based on the respective risk category assigned to each of the at least one parameter.

According to various embodiments, assigning the respective risk category to each of the at least one parameter based on the determined value of the respective parameter may include determining if the determined value of the respective parameter matches a corresponding reference value or lies within a corresponding reference value range, for the respective parameter associated with the respective risk category.

According to various embodiments, the reference value or reference value range for the respective parameter, may be established on the basis of a database comprising information of the risk of myopia onset or progression over the timeframe, for individuals within a population sample.

According to various embodiments, determining the subject's risk of the onset or progression of myopia over the timeframe, based on the respective risk category assigned to each of the at least one parameter may include determining, for each risk category of the plurality of predetermined risk categories, the number of parameters among the at least one parameter, to which the respective risk category has been assigned, and may further include determining, as the subject's risk of the onset or progression of myopia over the timeframe, that risk category of the plurality of predetermined risk categories, that has been assigned to a highest number of parameters.

According to various other embodiments, determining the subject's risk of the onset or progression of myopia over the timeframe, based on the respective risk category assigned to each of the at least one parameter may include assigning, a weight to each one of the plurality of predetermined risk categories, and determining, for each risk category of the plurality of predetermined risk categories, the number of parameters among the at least one parameter, to which the respective risk category has been assigned. The method may further include calculating, for each risk category of the plurality of predetermined risk categories, a score by multiplying the weight assigned to the respective risk category with the number of parameters, to which said risk category has been assigned, calculating, a final score by summing the scores calculated for the plurality of predetermined risk categories, and determining, the subject's risk of the onset or progression of myopia over the timeframe based on the final score.

According to various embodiments, determining the subject's risk of the onset or progression of myopia over the timeframe based on the final score may include, comparing the final score with at least one first predetermined threshold value.

According to various embodiments, the one or more predetermined risk categories may include: a low-risk category, a moderate-risk category, and a high-risk category.

According to various embodiments, determining the subject's risk of the onset or progression of myopia over the timeframe, based on the determined value of the at least one parameter, may be established based on at least one predictive model based on a machine learning algorithm, which provides a relationship between the risk of myopia onset or progression and the at least one parameter.

According to various embodiments, determining the subject's risk of the onset or progression of myopia over the timeframe, based on the determined value of the at least one parameter may include entering the determined value of the at least one parameter into the at least one predictive model based on the machine learning algorithm, calculating a value of a risk ratio, using the at least one predictive model based on the machine learning algorithm, and determining the subject's risk of the onset or progression of myopia over the timeframe based on the calculated value of the risk ratio. The calculated value of the risk ratio may provide a probability indicative of the subject's risk of the onset or progression of myopia over the timeframe, and may be carried out using the at least one predictive model based on the machine learning algorithm.

According to various embodiments, determining the subject's risk of the onset or progression of myopia over the timeframe based on the risk ratio may include, comparing the calculated value of the risk ratio with a second predetermined threshold value.

According to various embodiments, the at least one parameter associated with vision condition of said subject, may include a first parameter indicating a near refraction of said subject, and a second parameter indicating a near refraction sensitivity of said subject. The at least one parameter associated with vision condition of said subject, may also include a third parameter indicating a far refraction of said subject and a fourth parameter indicating a far refraction sensitivity of said subject. A fifth parameter indicating an overlap degree of a far comfort range and a near comfort range of said subject may be determined. Determining the value of the fifth parameter may include determining the far comfort range and the near comfort range based on the determined values of the first to fourth parameters, determining the overlap degree of the far and near comfort ranges, and assigning a value to the fifth parameter based on the determined overlap degree of the far and near comfort ranges.

According to various embodiments, determining the risk of the onset or progression of myopia may include determining a likelihood of progression to a higher degree of myopia, and/or a rate of progression of myopia.

According to various embodiments, the timeframe may be no more than about 6 months, about 1 year, about 2 years, about 3 years, about 4 years, or no more than about 5 years.

A second aspect of the disclosure concerns a system for determining a risk of an onset or progression of myopia over a timeframe. The system includes means for determining a value of at least one parameter associated with vision condition of a subject, said at least one parameter comprising a sensitivity parameter of said subject, said sensitivity parameter being relative to the sensitivity of said subject to a variation of at least one dioptric optical feature of at least one ophthalmic lens placed in front of at least one eye of said subject. The system further includes means for determining the subject's risk of the onset or progression of myopia over the timeframe, based on the determined value of the at least one parameter.

According to various embodiments, at least one of the means for determining the value of the at least one parameter, and the means for determining the subject's risk of the onset or progression of myopia, may include a circuit. In some embodiments, the means for determining the value of the at least one parameter, and the means for determining the subject's risk of the onset or progression of myopia, may be the same circuit.

According to various embodiments, the system may further include a computer program product including instructions to cause a computing system of the second aspect to execute the steps of the method of the first aspect.

## BRIEF DESCRIPTION OF THE DRAWINGS

The invention will be better understood with reference to the detailed description when considered in conjunction with the non-limiting examples and the accompanying drawings, in which:

- FIG. 1 shows an exemplary schematic illustration of method 100 for determining a risk of an onset or progression of myopia over a timeframe, in accordance with various embodiments;
- FIG. 2 shows an example of a subject's certitude probability function 200, which is used to determine a value of the subject's sensitivity parameter SRx, in accordance with various embodiments;
- FIG. 3 shows an exemplary schematic illustration of method 300 for determining a value of a fifth parameter, indicating an overlap degree of the far comfort range and the near comfort of the subject, in accordance with various embodiments;
- FIG. 4, 5A and 5B show exemplary schematic illustrations of methods 400, 500A, 500B for determining the subject's risk of the onset or progression of myopia over the timeframe, based on the determined value of the at least one parameter, in accordance with various embodiments;

- FIG. 6 shows an exemplary schematic illustration of method 600 for determining the subject's risk of myopia onset or progression over the timeframe, based on the determined value of the at least one parameter, in accordance with various embodiments;
- FIG. 7 shows an example of a system 700 for determining a risk of an onset or progression of myopia over a timeframe, in accordance with various embodiments;
- FIGS. 8A and 8B show tables of an example of a use condition of methods 400, 500A and 500B, to determine the subject's risk of myopia onset or progression over a timeframe, in accordance with various embodiments; and
- FIGS. 9A to 9C show tables of another example of a use condition of according to methods 300, 400, 500A and 500B, to determine the subject's risk of myopia onset or progression over a timeframe, in accordance with various embodiments.

## DETAILED DESCRIPTION

The following detailed description refers to the accompanying drawings that show, by way of illustration, specific details and embodiments in which the disclosure may be practiced. These embodiments are described in sufficient detail to enable those skilled in the art to practice the disclosure. Other embodiments may be utilized and structural, and logical changes may be made without departing from the scope of the disclosure. The various embodiments are not necessarily mutually exclusive, as some embodiments can be combined with one or more other embodiments to form new embodiments.

The disclosure illustratively described herein may suitably be practiced in the absence of any element or elements, limitation or limitations, not specifically disclosed herein. Thus, for example, the terms "comprising", "including," containing", etc. shall be read expansively and without limitation. The word "comprise" or variations such as "comprises" or "comprising" will accordingly be understood to imply the inclusion of a stated integer or groups of integers but not the exclusion of any other integer or group of integers. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the disclosure. Thus, it should be understood that although the present disclosure has been specifically described in exemplary embodiments and optional features, modification and variation of the disclosure embodied herein may be resorted to by those skilled in the art.

Features that are described in the context of an embodiment may correspondingly be applicable to the same or similar features in the other embodiments. Features that are described in the context of an embodiment may correspondingly be applicable to the other embodiments, even if not explicitly described in these other embodiments. Furthermore, additions and/or combinations and/or alternatives as described for a feature in the context of an embodiment may correspondingly be applicable to the same or similar feature in the other embodiments.

In the context of various embodiments, the articles "a", "an" and "the" as used with regard to a feature or element include a reference to one or more of the features or elements. As used herein, the term "and/or" includes any and all combinations of one or more of the associated listed items.

The reference signs included in parenthesis in the claims are for ease of understanding of the disclosure and have no limiting effect on the scope of the claims.

According to various embodiments, the term "myopia", as used herein, may refer to a refractive error of a subject, and may be measured in diopters (D). The term myopia may therefore include amongst others, axial myopia attributed to an increase in the eye's axial length; refractive myopia attributed to the condition of the refractive elements of the eye; curvature myopia attributed to excessive, or increased, curvature of one or more of the refractive surfaces of the eye, especially the cornea, for example, due to high corneal and lenticular power in a subject with Cohen syndrome; and index myopia attributed to variation in the index of refraction of one or more of the ocular media.

According to various embodiments, the term "risk", as used herein, may refer to a likelihood, probability or possibility of an occurrence of a situation, for example, an onset or progression of myopia for a subject over a timeframe. Accordingly, the term "determining a risk" may refer to a process or methods to determine or predict the likelihood or probability of an onset or progression of myopia for the subject over a timeframe. According to various embodiments, determining the subject's risk of the onset or progression of myopia over the timeframe may be for one eye, or for both eyes of the subject.

According to various embodiments, the term "onset", as used herein, may refer to the first occurrence or appearance of symptoms associated with myopia, such as reduced visual acuity and intermittent blurred vision. Within the context of the disclosure, the onset of myopia over a timeframe may refer to the first occurrence of such symptoms for an emmetropic, e.g. pre- or non-myopic subject over a timeframe.

According to various embodiments, the term "progression", as used herein, may refer to the worsening of myopia for a myopic subject over a timeframe. In other words, the subject already has myopia. In particular, the term progression may refer to a speed or rate of progression of myopia for a myopic subject over a timeframe, for example, the progression from a spherical measurement of about -2.00 D to about -3.00 D, or to about -6.00 D, over a period. In another example, the term progression may refer to the subject's progression from low myopia to moderate myopia or high myopia, or from moderate myopia to high myopia.

According to various embodiments, the term "low myopia", as used herein, may correspond to a spherical equivalent of -3.00 D or less negative; the term "moderate myopia", as used herein, may correspond to a spherical equivalent between -3.00 D to -6.00 D; and the term "high myopia" may correspond to a spherical equivalent of -6.00 D or more negative. The spherical equivalent may be a function of the sphere power, e.g. myopia, and the cylinder power, e.g. astigmatism. For example, the spherical equivalent may be equal to the sphere power + 0.5(cylinder power).

According to various embodiments, the term "value", as used herein, may refer to as a numerical value, for example, a discrete numerical value or as a range of numerical values. Alternatively, or in addition, the term "value" may refer to a text, e.g. a string of text, for example, *"yes", "no", "overlap"* or *"no overlap"*.

According to various embodiments, the term "parameter(s) associated with vision condition", as used herein, may refer to parameters, e.g. factors, related to the visual condition or system for a subject. Within the context of the disclosure, the term parameter(s) associated with vision condition may include parameters which influence or causes the onset or progression of myopia over a timeframe, for an emmetropic or myopic subject respectively. For example, the parameter(s) associated with vision condition may include genetic factors, environmental factors, or a combination thereof.

According to various embodiments, the term "subject", as used herein, may refer to an emmetropic, i.e. non- or pre-myopic individual, and accordingly determining a risk may refer to determining a risk of an onset of myopia over a timeframe for said emmetropic individual. According to various other embodiments, the term "subject", as used herein, may refer to a myopic or ametropic individual, and accordingly determining a risk may refer to the determining a risk of progression of myopia over a timeframe for said myopic individual.

According to various embodiments, the term "sensitivity parameter", as used herein, may refer to a value of the subject's sensitivity parameter, e.g. personalized sensitivity parameter. In some embodiments, the sensitivity parameter includes a specific sensitivity parameter. For example, the specific sensitivity parameter may be selected from: sphere sensitivity to the variation of sphere of at least one ophthalmic lens for at least one of the eyes of the subject; cylinder and/or axis sensitivity to the variation of cylinder power and/or axis of at least one ophthalmic lens for at least one of the eyes of the subject; sphere binocular sensitivity to a variation in binocular balance of at least one ophthalmic lens for at least one of the eyes of the subject; addition sensitivity to the variation in the addition of at least one ophthalmic lens placed in front of at least one eye of the subject. In some other embodiments, the sensitivity parameter includes a global sensitivity parameter which refers to an average value, or weighted average value of several single values of specific sensitivity parameters.

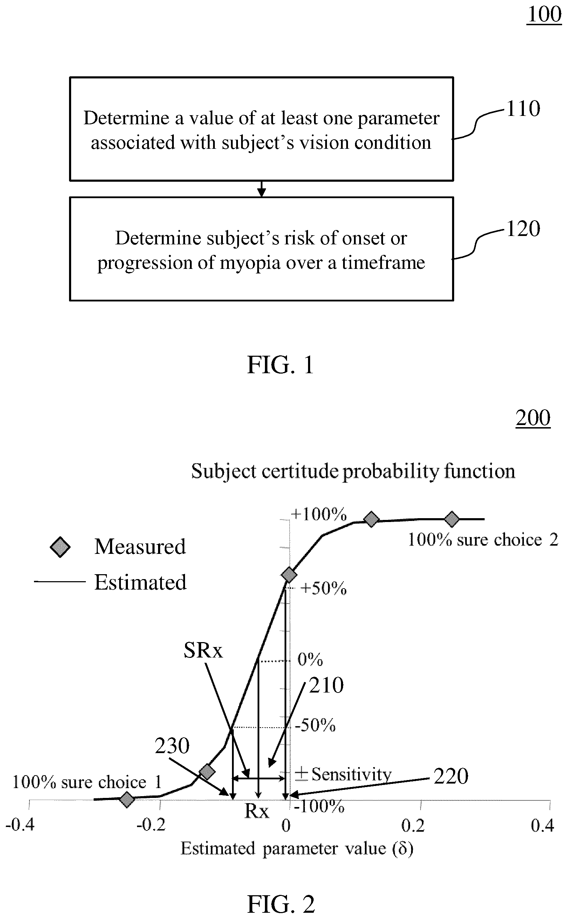

FIG. 1 shows a schematic illustration of method 100 for determining a risk of an onset or progression of myopia over a timeframe, by way of example. Method 100 includes at step 110, determining a value of at least one parameter associated with the vision condition of a subject. The at least one parameter associated with the vision condition of a subject includes the sensitivity parameter SRx of said subject, the sensitivity parameter SRx being relative to the sensitivity of said subject to a variation of at least one dioptric optical feature of at least one ophthalmic lens placed in front of at least one eye of said subject. Method 100 further includes at step 120, determining the subject's risk of the onset or progression of myopia over the timeframe based on determined value of the at least one parameter.

FIG. 2 shows an example of a subject's certitude probability function 200, which is used to determine a value of the subject's sensitivity parameter SRx. The subject's certitude probability function 200 may be represented as a graph of the certitude of the choices made by the subject, as a function of the optical feature value of the lens(es) placed in front of the eye(s) of the subject. The subject's certitude probability function 200 is defined as the product of the subject's answers by the probability of certitude as a function of the estimated parameter values, and may be obtained based on the subject's choices relative to the variation of at least one dioptric optical feature of at least one ophthalmic lens placed in front of at least one eye of the subject. In other words, the subject's certitude probability function 200 reflects the certainty of the subject when making a choice between the two visual states or between two different lenses placed successively in front of his eye. This may be determined from a standard eye examination, e.g. Jackson Cross Cylinder procedure with or without fixed Jackson cross cylinders, duo-chrome test, perceptual appreciation test, binocular balance test, or other eye examination methods known to those skilled in the art.

For example, the subject may be asked to choose between two visual states or two different lenses placed successively in front of his eye, e.g. choice 1 and choice 2 on the certitude probability function 200. The 0 % certitude value is the value obtained when the subject is unable to choose between the two visual states or the two lenses, for example, when the subject is unable to perceive any difference in the quality of the image seen in each visual state, e.g. choice 1 or choice 2. This may be represented by the value "0", "don't know" or "null" during the eye examination. The -100 % certitude value is the value obtained when the subject is certain that the first of the two visual states, or that the first lens, e.g. choice 1, provides a better quality of the image seen. This may be represented by value "-1" during an eye examination. The +100 % certitude value is the value obtained when the subject is certain that the second visual state, or the second lens, e.g. choice 2 provides a better quality of the image seen, and may be represented by value "+1" during an eye examination. Any uncertain answer may also be represented by a value between "-1 or +1" depending on the estimated or measured certitude.

The subject's certitude probability function 200 is then calculated by averaging all the answers estimated for a particular optical feature value, and then interpolated to any non-estimated value. An insensitive zone 210 may then be defined, corresponding to the values of the optical feature of the lens of which probability of certitude is comprised in a predetermined range of incertitude. In some embodiments, the range of incertitude are the ranges [-70 %, 70 %], [-60 %, 60 %] or preferably [-50 %, 50 %] or [-40 %, 40 %]. In other words, the insensitive zone 210 is defined and limited by a high level 220 and a low level 230 that are the 2 bounds for which the probability of certitude is below the predetermined range, e.g. [-50 %, 50%].

The value of the sensitivity parameter SRx may then be defined to be equal to half of the insensitive zone 210 size, as represented by Equation 1:

$$SRx = \frac{[High\ level - Low\ level]}{2} \qquad (1)$$

That is to say, the value of the sensitivity parameter SRx may correspond to the value of certainty at 0 % of the subject's probability certitude function 200.

In other words, the insensitive zone 210 may correspond to the range of values of the optical feature that could be acceptable for the subject, or which the subject cannot decide which lens provides better vision. The value of the sensitivity parameter SRx may then be determined from the subject's certitude probability function 200 as the width of half of the insensitive zone 210 of the optical feature variation, for which the subject is insensitive to the change in the value of the optical feature of the lens placed in front of the subject's eye.

Methods for determining the value of the sensitivity parameter SRx are known from document WO 2020/016398. Methods for determining the certitude probability function are known from document WO 2018/015381.

Lag of accommodation, e.g. the extent to which the eye(s) of the subject fails to focus accurately in response to a visual stimulus, has been shown to strongly increase after the onset of myopia and myopic subjects have been shown to have a lower sensitivity to blur than emmetropes (Rosenfield M. et al. Optom. Vis. Sci. 1999, 76(5): 303-307). Currently, optometric measurement methods such as the *"les optimales convexes et concaves"* of the *"variations dioptriques"* provide an estimation of the sensitivity parameter SRx. However, these optometric measurement methods include additional tests with complex instructions which may not be fully understood by the subject, in particular, a child. In accordance with various embodiments, a subject's lag of accommodation may be related to the subject's insensitive zone 210 on the subject's certitude probability function 200 and as such, the value of the subject's sensitivity parameter SRx.

Advantageously, determining the value of the subject's sensitivity parameter SRx based on the subject's certitude probability function 200 provides a direct and simple way of determining the sensitivity parameter SRx in a precise and accurate manner without the need for additional measurements or instructions that may be complex to the subject, in particular, a young child.

Referring again to FIG. 1, step 110 of method 100 may further include determining the value of at least one parameter associated with vision condition of the subject. In accordance with various embodiment, the at least one parameter may further include: a dioptric optical parameter, a parameter relating to the subject's lifestyle, activity or behavior, a parameter relating to the subject's genetic history, an optical biometric parameter, and/or a parameter relating to personal information about the subject.

According to various embodiments, the subject's dioptric optical parameter may include an objective measurement of a subject's refractive error, e.g. spherical power obtained during an eye examination. Said parameter may be obtained using any ophthalmic testing device, e.g. phoropter, refractor, autorefractor and/or a retinoscope. As a further example, the dioptric optical parameter may include or be the rate of myopia progression in a previous timeframe, e.g. previous year, for a myopic subject, for instance from low myopia to moderate myopia or high myopia in the previous year.

According to various embodiments, the parameter relating to the subject's lifestyle, activity or behavior may include the subject's level of exposure to sufficient light. For example, the parameter relating to the subject's lifestyle, activity or behavior may include environmental factors such as the subject's level of physical activity, duration of near work, and/or education level. In some embodiments, the parameter relating to the subject's lifestyle, activity or behavior may be the duration the subject spends outdoors per day, for example, engaging in physical exercise or in outdoor play sessions. In some other embodiments, the parameter relating to the subject's lifestyle, activity or behavior may be the average exposure to sunlight or natural light per day, intensity of natural light exposure, average UV index, latitude, or any combination thereof. Without wishing to be bound by theory, it has been observed that insufficient light exposure, insufficient time spent outdoors per day, a prolonged duration spent in near vision, and higher education levels increases the risk of the onset or progression of myopia over a timeframe. Accordingly, the duration of time spent outdoors has been associated with a protective effect against the development of myopia which may be due, at least in part, to the effect of long hours of exposure to daylight on the production and the release of retinal dopamine.

According to various embodiments, the parameter relating to the subject's genetic history may include or be the number of myopic parents the subject has. In another example, the parameter relating to the subject's genetic history may include the number of myopic siblings the subject has, or be the number of myopic extended family members, e.g. grandparents, the subject has. Without wishing to be bound by theory, a risk for myopia onset and progression over a timeframe may be inherited from one's parents, and a family history of myopia has been shown to be correlated with an increased risk of myopia onset or progression over a timeframe.

According to various embodiments, the optical biometric parameter may include an objective measurement of a subject's axial length (in mm), vitreous chamber depth (in mm), choroidal thickness (in $\mu$m), and may further include other corneal characteristics, including corneal hysteresis. Said parameter may be obtained during an eye examination and using any ophthalmic testing device, e.g. phoropter, refractor, autorefractor and/or a retinoscope.

According to various embodiments, the parameter relating to personal information about the subject may include or be the subject's gender, age, ethnicity, or combinations thereof. It is further envisioned that the parameter relating to the subject's personal information may also include underlying medical conditions such as diabetes, childhood arthritis, uveitis, and systemic lupus erythematosus. It has been observed that myopia is more common in individuals, e.g. children with said medical conditions.

Thus, step 110 of method 100 may include determining the value of the at least one parameter associated with vision condition of the subject, which may include the subject's sensitivity parameter SRx, the subject's dioptric optical parameter, the parameter relating to the subject's lifestyle, activity or behavior, the parameter relating to the subject's genetic history, the optical biometric parameter and/or the parameter relating to personal information about the subject. In some embodiments, step 110 of method 100 includes determining the value of all the aforementioned parameters associated with vision condition of the subject.

According to various embodiments, the at least one parameter associated with vision condition of the subject may also include a fifth parameter, indicative of an overlap degree of subject's far comfort range and near comfort range.

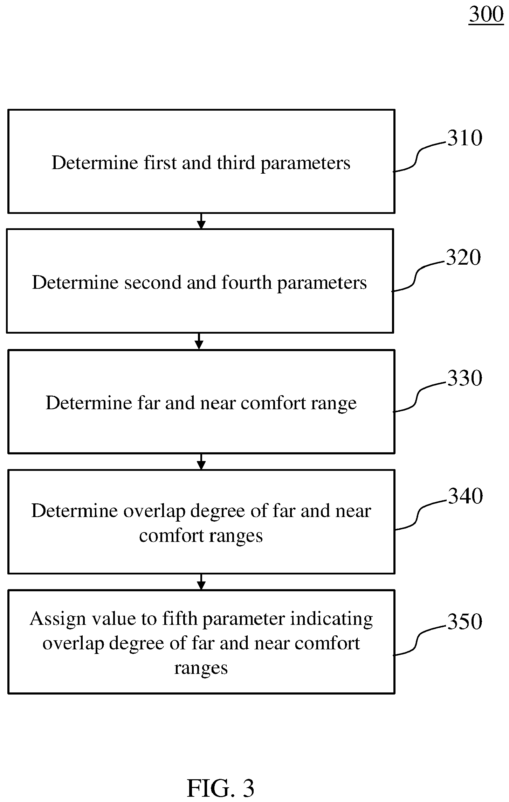

FIG. 3 shows a schematic illustration of method 300 for determining a value of a fifth parameter, indicating an overlap degree of the far comfort range and the near comfort of the subject, by way of example. At step 310, method 300 may include determining a first parameter indicating a near refraction of the subject. For example, the first parameter, e.g. subject's near refraction, may correspond to the subject's near prescription and may be obtained during the subject's eye examination. Step 310 may also include determining a third parameter indicating a far refraction of the subject. For example, the third parameter, e.g. subject's far refraction, may correspond to the subject's far prescription, obtained during the subject's eye examination.

Step 320 of method 300 may include determining a second parameter indicating a near refraction sensitivity of the subject, and may further include determining a fourth parameter indicating a far refraction sensitivity of the subject. The near and far refraction sensitivity may be determined using the subject's near and far certitude probability functions, respectively. The method to determine the subject's near and far certitude probability functions may be the same as the method used to obtain the subject's sensitivity parameter SRx, which has been explained above with reference to FIG. 2. Briefly, the subject's near and far certitude probability function may be calculated by averaging the answers estimated for a particular optical feature value, e.g. near and far prescription, respectively. The near and far prescription certitude probability function may then be interpolated to any non-estimated value. A near and far insensitive zone may be defined on the subject's near and far certitude probability function, respectively. The near and far insensitive zones may correspond to the values of the optical feature of the lens of which probability of certitude is comprised in a predetermined range of incertitude, e.g. [-50 %, 50 %]. In other words, the near and far refraction insensitive zone may be defined and limited by a high level and a low level on the near or far certitude probability function, respectively, that are the 2 bounds for which the probability of certitude is below the predetermined range, e.g. [-50 %, 50%]. The value of the near or far refraction sensitivity, e.g. second and fourth parameter, respectively, may then be defined to be equal to half of the near or far insensitive zone size. Alternatively, the near and far refraction sensitivity, e.g. second and fourth parameters, may be defined as being both equal to the global sensitivity parameter, which may be calculated from various specific sensitivity parameters SRx measured in either, e.g. near or far conditions, or both, e.g. near and far conditions.

In some embodiments, either the near refraction sensitivity, e.g. second parameter, or the far refraction sensitivity, e.g. fourth parameter may be measured, and either parameter may be used to determine the subject's risk of the onset or progression of myopia over the timeframe, e.g. via a predictive model which provides a relationship between the risk of myopia onset or progression and the second or fourth parameter. Accordingly, the time taken to perform the measurement and obtain the second or fourth parameter may be advantageously be shortened. For example, with a small child, the time taken to perform the measurement may be a relevant criterion. In some other embodiments, both the near and far refraction sensitivity, e.g. second and fourth parameters may be measured and used to determine the subject's risk of the onset or progression of myopia over the timeframe.

At step 330, method 300 may include determining a near comfort range which may be determined based on the subject's near prescription, e.g. first parameter, and near refraction sensitivity, e.g. second parameter, obtained at steps 310 and 320, respectively. The near comfort range may be calculated according to Equation 2:

$$Near\ comfort\ range = near\ prescription\ \pm (K\_near \times near\ refraction\ sensitivity) \text{-----} \quad (2)$$

In Equation 2, the coefficient ($K\_near$) may represent a subject specific, e.g. personalized coefficient. An associated confidence interval for the near comfort range is given by $2 \times K\_near \times near\ refraction\ sensitivity$. The coefficient $K\_near$ and associated confidence interval may depend on the width of the function, e.g. Gaussian function, of the near refraction sensitivity, e.g. second parameter. In some embodiments, a 95 % confidence interval may be used. In this case, $K\_near$ may be 1.96 under the assumption of a Gaussian distribution for the near refraction sensitivity. Alternatively, other confidence intervals, such as 50 %, 90 %, 98 % or 99 % may be used.

Step 330 of method 300 may also include determining the far comfort range, in a similar manner as that of the near comfort range. The far comfort range may be based on the far prescription, e.g. third parameter, and far refraction sensitivity, e.g. fourth parameter obtained at steps 310 and 320, respectively. The far comfort range may be calculated according to Equation 3:

$$Far\ comfort\ range = far\ prescription\ \pm (K\_far \times far\ refraction\ sensitivity) \quad (3)$$

In Equation 3, the coefficient ($K\_far$) may represent a subject specific, e.g. personalized coefficient. An associated confidence interval for the far comfort range is given by $2 \times K\_far \times far\ refraction\ sensitivity$. The coefficient $K\_far$ and associated confidence interval may depend on the width of the function, e.g. Gaussian function, of the far refraction sensitivity, e.g. fourth parameter. In some embodiments, a 95 % confidence interval may be used. In this case, $K\_far$ may be 1.96 under the assumption of a Gaussian distribution for the near refraction sensitivity. Alternatively, other confidence intervals, such as 50 %, 90 %, 98 % or 99 % may be used.

Step 340 of method 300 may include determining an overlap degree of the near and far comfort ranges. In other words, step 340 includes determining if the far comfort range overlaps with the near comfort range. In some embodiments, if it is determined that the far and near comfort ranges overlap, step 340 may further include determining if the far refraction, e.g. third parameter determined at step 310, lies within the near comfort range, determined at step 330.

At step 350, method 300 may include assigning a value to a fifth parameter indicating the overlap degree of the near and far comfort ranges, which may be determined at step 340. For example, values that may be assigned to the fifth parameter may include but are not limited to: *"no overlap", "overlap", "overlap but far refraction not in near comfort zone"*. Alternatively, the value that may be assigned to the fifth parameter may include the percentage overlap degree of the near and far comfort ranges.

As indicated above, the at least one parameter associated with vision condition may include the fifth parameter indicating the overlap degree of a far comfort range and near comfort range, which may be determined using method 300. Without wishing to be bound by theory, it has been observed that if the far and near comfort ranges overlap, the subject has a low risk of myopia onset or progression over a timeframe. Conversely, if the far and near comfort ranges do not overlap, the subject is at significant risk of developing myopia, for example, at significant risk of myopia onset for a pre-myopic subject over a timeframe, or at a significant risk of progression to a higher degree of myopia over a timeframe (Drobe B. et al. Ophthal. Physiol. Opt. 1995, 15, 375-378). Thus, the fifth parameter may provide an additional factor to improve the prediction power of existing or new myopia onset or progression models.

In non-limiting embodiments, examples of parameters associated with the subject's vision conditions may further include parameters relating to the visual aptitudes or to the visual deficiency of the subject, for example, hypermetropia, astigmatism, presbyopia, or any other visual disease such as ocular diseases that result in visual issues such as myopic macular degeneration, retinal detachment and glaucoma.

Step 120 of FIG. 1 includes determining the subject's risk of onset or progression of myopia over a timeframe based on the determined value of the aforementioned at least one parameter associated with vision condition of the subject.

According to various embodiments, determining the subject's risk of the onset or progression of myopia may include determining a likelihood of progression to a higher degree of myopia within a timeframe. The likelihood of progression may be expressed in the form of the risk ratio, e.g. percentage or ratio, or may be expressed as categorical risk, e.g. low-risk, moderate- risk or high-risk of progressing to a higher degree of myopia. In some embodiments, the higher degree of myopia may include low myopia, moderate myopia, or high myopia. For example, for an emmetropic subject, determining the subject's risk of the onset or progression may include determining the subject's risk of progression from being non-myopic to low myopia over a timeframe. As a further example, for the emmetropic subject, determining the subject's risk may include predicting the age at which the subject may develop myopia. In another example, for a myopic subject, determining the subject's risk of the onset or progression may include determining the subject's risk of progression to a higher degree of myopia, for instance, from low myopia to moderate myopia or to high myopia, within a timeframe. As a further example, for the myopic subject, determining the subject's risk includes predicting the age at which the subject progresses from low myopia to moderate myopia or high myopia.

According to various embodiments, determining the subject's risk of the onset or progression of myopia may include determining a rate of progression of myopia. The rate of progression may include predicting a change in the degree of myopia within a timeframe. The predicted change in degree of myopia may be about 1 D or less, about 1 to 2 D, about 2 to 3 D, about 3 to 4 D, about 4 to 5 D, about 5 to 6 D, about 6 to 7 D, about 7 to 8 D, about 8 to 9 D, or about 9 to 10 D.

According to various embodiments, determining the subject's risk of the onset or progression of myopia is over, i.e. relative to, a timeframe. The timeframe for predicting the subject's risk of the onset or progression of myopia may be the same timeframe for which the database comprising information on the risk of myopia onset or progression is established on, e.g. timeframe of the longitudinal study, as will be explained below. For example, the timeframe may include but is not limited to about 6 months, about 1 year, about 2 years, about 3 years, about 4 years, about 5 years, about 8 years, or about 10 years. In some embodiments, the timeframe is no more than 5 years. That is to say, the probability of the subject's progression to a higher degree of myopia, and/or a rate of progression of myopia is established relative to the indicated timeframe.

Referring again to step 120 of FIG. 1, determining the subject's risk of the onset or progression of myopia over the timeframe, based on the determined value of the at least one parameter, may be established based on at least one predictive model which provides a relationship between the risk of myopia onset or progression and the at least one parameter, in accordance with various embodiments. In other words, the subject's risk of the onset or progression of myopia over the timeframe may be implemented and determined using a predictive model, which may be implemented using a machine learning algorithm. For example, one or more neural networks may be trained by inputting a series of determined values of the parameter associated with vision condition of numerous individuals, e.g. population sample, which may have the same or similar profile, e.g. sensitivity parameter SRx, age, genetic history, dioptric optical parameter, and building a correlation table or any database containing information on the relationship between the risk of myopia onset or progression and the at least one parameter associated with vision condition. In an embodiment, said database or correlation table may include or be the longitudinal study which established the correlation of the risk of myopia onset or progression over a timeframe and the at least one parameter associated with vision condition.

According to various embodiments, the machine learning algorithm may be selected from the group consisting of supervised, semi-supervised and unsupervised learning, for example: a support vector machine (SVM), a Naive Bayes classification, a random forest, an artificial neural network, a decision tree, a K-means, learning vector quantization, self-organizing maps, graphical models, preferably, regression algorithms, e.g. linear, multi-variate, logistic, association rule learning, deep learning, dimensionality reduction and ensemble selection algorithms.

In a non-limiting embodiment, it is envisioned that the self-reported parameter may be amounted for by the at least one predictive model. For example, self-reported parameters include data from social networks or the genetic risk score related to a visual deficiency or disease. Such self-reported parameters will in turn modify the prediction model.

Methods for building the at least one predictive model based on the machine learning algorithm may be known from WO 2020/126513.

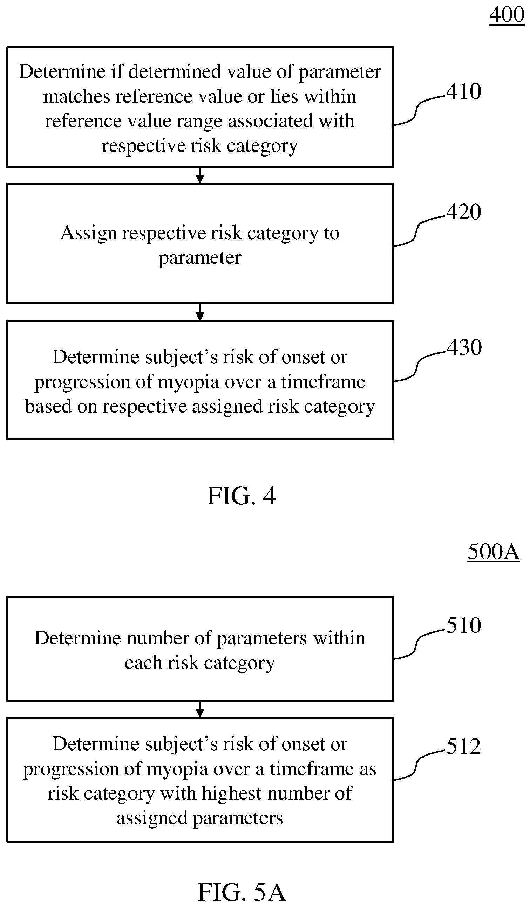

FIG. 4 to 5B show schematic illustrations of methods 400, 500A and 500B for determining the subject's risk of the onset or progression of myopia over the timeframe, based on categorical risk, in accordance with various embodiments of the invention. In other words, the predictive model for determining the subject's risk of myopia onset or progression over a timeframe may include or be a categorical model. Specifically, the predictive model may include one or more predetermined risk categories, including at least, a low-risk category, a moderate-risk category, and a high-risk category.

According to various embodiments, each of the predetermined risk categories includes a reference value, or reference value range associated with the respective parameter. For example, the low-risk, moderate-risk, and high-risk categories each include a reference value or reference value range connected to the at least one parameter associated with vision condition of the subject, e.g. the sensitivity parameter SRx, the dioptric optical parameter, the parameter relating to the subject's lifestyle, activity or behavior, the parameter relating to the subject's genetic history, the optical biometric parameter, the parameter relating to personal information about the subject, and/or the fifth parameter indicating the overlap degree of the subject's near and far comfort zone.

According to various embodiments, the term "individuals within a population sample", as used herein, may refer to a group, e.g. any number of individuals who may share at least one common characteristic with each other. For example, the individuals within the population sample may share a same or similar dioptric optical parameter, age, ethnicity, genetic history, lifestyle, activity or behavior, optical biometric parameter, sensitivity parameter SRx, and/or parameter including information on the overlap degree of the near and far comfort ranges.

According to various embodiments, the reference value or reference value range may be established on the basis of a database comprising information of the risk of myopia onset or progression over the timeframe, for individuals within a population sample. For example, the database may be established based on longitudinal studies of the risk of myopia onset or progression over a period of time, for individuals within a population sample, e.g. children under the age of 12 years old. The database may thus include information linking the risk of myopia onset or progression over the timeframe with a specific parameter associated with vision condition, for instance, the sensitivity parameter SRx, the fifth parameter indicating the overlap degree of the subject's near and far comfort zones, the dioptric optical parameter, the parameter relating to the individual's genetic history, the optical biometric parameter, and the parameter relating to the individual's lifestyle, activity or behavior.

FIG. 4 shows a schematic illustration of method 400 for determining the subject's risk of the onset or progression of myopia over the timeframe, based on the determined value of the respective parameter, by way of example. At step 410, method 400 includes determining if the determined value of the respective parameter, obtained at step 110 of FIG. 1, matches a corresponding reference value or lies within a corresponding reference value range, for the respective parameter associated with the respective risk category. For example, step 410 may include determining if the determined value of the sensitivity parameter SRx, is equal to a corresponding reference value or falls within a corresponding reference value range, for the reference value range of the sensitivity parameter SRx for each of the low-risk category, the moderate-risk category, and the high-risk category. Said reference value, or reference value range may be established from the database comprising information of the risk of myopia onset or progression over the timeframe for individuals within the population sample. In other words, the low-risk, moderate-risk, and high-risk categories each include a reference value or reference value range for each parameter associated with vision condition, and step 410 includes determining if the determined value of the parameter associated with vision condition of the subject is equal to the reference value, or falls within the reference value range.

Step 420 of method 400 may include assigning to each of the at least one parameter, a risk category selected from a plurality of predetermined risk categories, based on step 410, which may include determining if the determined value of the respective parameter matches a corresponding reference value or lies with the corresponding reference value range for the respective parameter associated with the respective risk category. For example, if the determined value of the respective parameter matches the corresponding reference value or lies within the corresponding reference value range for the respective risk category, the parameter may be assigned to the respective risk category. That is to say, if the determined value of the parameter, e.g. sensitivity parameter SRx, is equal to or falls within the respective reference value range of the low-risk, moderate-risk, or high-risk categories, the parameter will be assigned to that category.

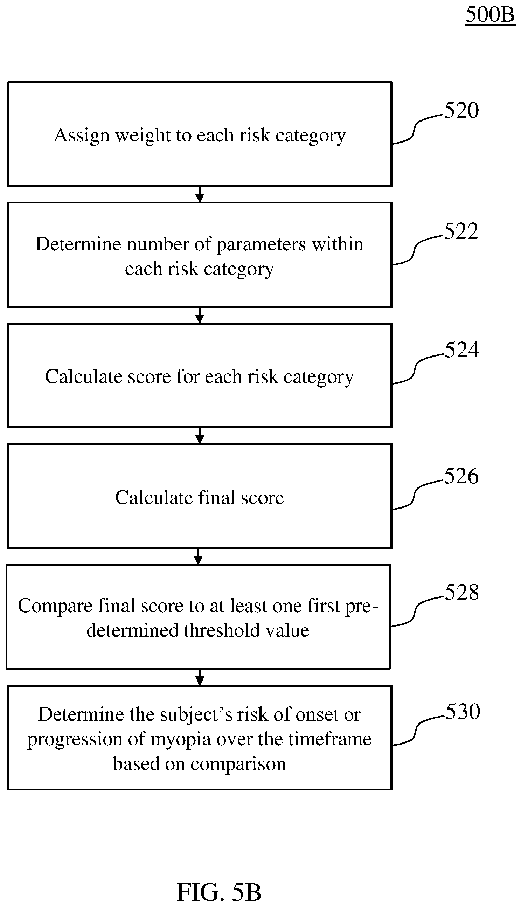

Step 430 of method 400 may include, determining the subject's risk of the onset or progression of myopia over the timeframe, based on the respective risk category assigned to each of the at least one parameter, as will be explained with reference to FIGS. 5A and 5B.

FIG. 5A shows a schematic illustration of an exemplary method 500A for determining the subject's risk of the onset or progression of myopia over the timeframe, based on the respective risk category assigned to each of the at least one parameter. In an embodiment, determining the subject's risk of the onset or progression of myopia over the timeframe may include, at step 510, determining for each risk category of the plurality of predetermined risk categories, the number of parameters among the at least one parameter, to which the respective risk category has been assigned. In other words, step 510 includes determining the total number of parameters within each of the low-risk, moderate-risk, and high-risk categories. For example, the predictive model may include instructions to calculate the sum of the, e.g.

total number of parameters within each of the low-risk, moderate-risk and high-risk categories.

Method 500A may further include, at step 512, determining, as the subject's risk of the onset or progression of myopia over the timeframe, that risk category of the plurality of predetermined risk categories, that has been assigned to a highest number of parameters. That is to say, step 512 includes comparing the total number of parameters within each of the predetermined risk categories, e.g. compare total number of parameters between each of the low-risk, moderate-risk and high-risk categories, and determining as the subject's risk of onset or progression of myopia over the timeframe, the specific risk category with the highest total number of parameters. For example, if the low-risk category includes 2 parameters that fall within said category, and the moderate-risk and high-risk categories each include 1 parameter, the subject's risk of the onset or progression of myopia over the timeframe may be determined as being of low-risk.

FIG. 5B shows a schematic illustration of another method 500B for determining the subject's risk of the onset or progression of myopia over the timeframe, based on the respective risk category assigned to each of the at least one parameter, by way of example. In another embodiment, determining the subject's risk of the onset or progression of myopia over the timeframe may include, at step 520, assigning a weight to each one of the plurality of predetermined risk categories. For example, a quantitative, e.g. numerical value may be assigned to each of the low-risk, moderate-risk, and high-risk categories. As a further example, the low-risk category may be assigned a weight of "0", the moderate-risk category assigned a weight of "1", and the high-risk category assigned a weight of "2".

Method 500B may also include at step 522, determining for each risk category of the plurality of predetermined risk categories, the number of parameters among the at least one parameter, to which the respective risk category has been assigned. Step 522 may be the same as step 510 of method 500A. Repeated descriptions are omitted for brevity. Briefly, step 522 includes determining the total number of parameters within each of the low-risk, moderate-risk, and high-risk categories.

Step 524 of method 500B may include, calculating, for each risk category of the plurality of predetermined risk categories, a score by multiplying the weight assigned to the respective risk category with the number of parameters, to which said risk category has been assigned. For example, the score for each of the low-risk, moderate-risk, and high-risk categories may be calculated by multiplying the weight assigned to the respective risk category (at step 520) by the total number of parameters assigned to the respective risk category (at step 522).

Step 526 of method 500B may then include calculating a final score by summing the scores calculated for the plurality of predetermined risk categories. For example, the final score is calculated by adding the individual scores determined at preceding step 524 for each of the low-risk, moderate-risk, and high-risk categories.

To determine the subject's risk of the onset or progression of myopia over the timeframe, method 500B may include, at step 528, comparing the final score to at least one first predetermined threshold value. The first predetermined threshold value may be established on the basis of the database including information on the risk of myopia onset or progression over the timeframe for individuals within the population sample. For example, the final score may be compared to the at least one first predetermined threshold value, e.g. for that of the low-risk category, moderate-risk category and high-risk category, and may include determining if the final score is equal to, greater than, or less than the at least one first predetermined threshold value.

Step 530 of method 500B may then include determining the subject's risk of myopia onset or progression over the timeframe based on the comparison of the final score to the at least one first predetermined threshold value. For example, if it is determined in preceding step 528 that the final score is equal to or greater than the first predetermined threshold value of the high-risk category, the subject's risk of myopia onset or progression over the timeframe may be determined as being of high-risk.

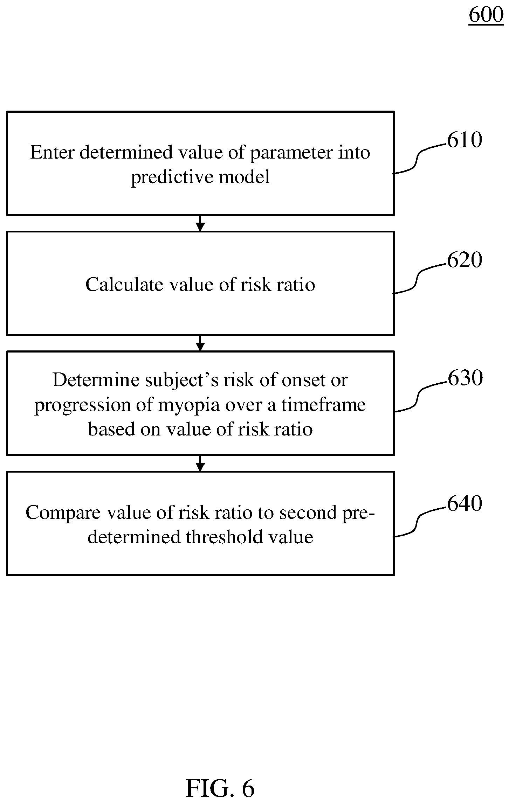

FIG. 6 shows a schematic illustration of method 600 for determining the subject's risk of myopia onset or progression over the timeframe based on the determined value of the at least one parameter, by way of example, and in accordance with various embodiments. Method 600 may include, at step 610, entering the determined value of the at least one parameter (at step 110) into the at least one predictive model based on the machine learning algorithm. For example, step 610 may include inputting amongst others, the determined value of the sensitivity parameter SRx, and/or the fifth parameter indicating the overlap degree of the subject's far and near comfort zones, into the predictive model based on the machine learning algorithm.

Method 600 may also include, at step 620, calculating a value of a risk ratio, using the at least one predictive model based on the machine learning algorithm. As mentioned above, the machine learning algorithm may include a correlation table or any database including information on the relationship between the risk of myopia onset or progression over the timeframe and the at least one parameter associated with vision condition. According to various embodiments, the value of the risk ratio may be calculated based on the machine learning algorithm. For example, the machine learning algorithm may include a multifactorial equation correlating the risk of myopia onset or progression over the timeframe and the respective at least one parameter associated with vision condition, for individuals within a population sample. Accordingly, the calculated value of the risk ratio determined at step 620, provides a probability indicative of the subject's risk of the onset or progression of myopia over the timeframe, and is carried out using the at least one predictive model based on the machine learning algorithm.

At step 630, method 600 may include determining the subject's risk of the onset or progression of myopia over the timeframe based on the calculated value of the risk ratio. For example, the calculated value of the risk ratio may be expressed as a risk percentage (%) that provides an indicative probability of the subject's risk of myopia onset or progression over the timeframe. As a further example, the risk ratio may be expressed as being a value ranging from 0 to 100, or alternatively from 0.0 to 1.0, over a timeframe.

In some embodiments, method 600 may include an additional step 640, which includes comparing the calculated value of the risk ratio with a second predetermined threshold value. The second predetermined threshold value may be established on the basis of the database including information on the risk of myopia onset or progression over the timeframe for individuals within the population sample. In an example, the second predetermined threshold value may be a threshold value that indicates that the subject may be at a moderate, or high risk of myopia onset or progression.

In accordance with various embodiments, the methods 100, 300, 400, 500A, 500B and 600 for determining the subject's risk of the onset or progression of myopia over the timeframe may be based on at least one predictive model based on a machine learning algorithm which provides a relationship between the risk of myopia onset or progression and the respective parameter, as explained above. In some embodiments, the risk may be determined as categorical risk, e.g. as low-risk, moderate-risk or high-risk. Alternatively, the risk may be determined as continuous risk, e.g. as a percentage or risk ratio. In some embodiments, the subject's risk of the onset or progression of myopia over the timeframe may include both categorical and continuous risk.

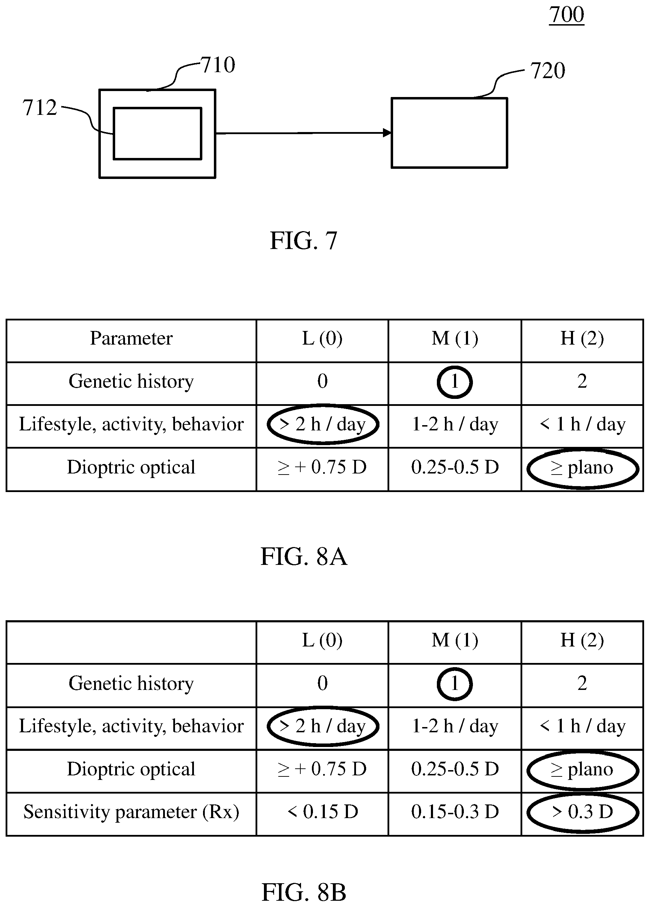

FIG. 7 shows an example of a system 700 for determining a risk of an onset or progression of myopia over a timeframe. System 700 includes means for determining a value of at least one parameter associated with vision condition of the subject 710, which may include at least one circuit, e.g. microprocessor(s). For example, the means for determining a value of at least one parameter associated with vision condition of the subject 710 may include any ophthalmic testing device, such as a computer-controlled machine used during an eye examination to provide an objective measurement of the subject's refractive error, e.g. phoropter, refractor, autorefractor and/or a retinoscope. For instance, the ophthalmic testing device may be used to determine the subject's dioptric optical parameter, the first parameter and third parameters indicating the near and far refraction, respectively, of the subject.

According to various embodiments, the means for determining a value of at least one parameter associated with vision condition of the subject 710 also includes determining the subject's sensitivity parameter SRx, said sensitivity parameter SRx being relative to the sensitivity of said subject to a variation of at least one dioptric optical feature of at least one ophthalmic lens placed in front of at least one eye of said subject. Thus, the at least one circuit may be configured, e.g. include instructions, to determine the value of sensitivity parameter SRx. In some embodiments, the at least one circuit may further determine the second parameter indicating the near refraction sensitivity of the subject, the fourth parameter indicating the far refraction sensitivity of the subject, and the fifth parameter indicating the overlap degree of the far and near comfort ranges of the subject. Methods for determining the value of the sensitivity parameter SRx, and the second, fourth and fifth parameters have been explained above and repeated descriptions are omitted for brevity.

According to various embodiments, means for determining a value of at least one parameter associated with vision condition of the subject 710 may also be configured to receive the other parameters associated with vision condition of the subject. For example, the means for determining a value of at least one parameter associated with vision condition of the subject 710 may include an interface 712 configured to receive the subject's input. As a further example, the interface 712 may be a display on the means for determining a value of at least one parameter associated with vision condition of the subject 710, e.g. microprocessor. As such, the subject (or third party) may input the other parameters associated with vision condition, for instance parameters relating to the subject's lifestyle, activity or behavior, parameters relating to the subject's genetic history, the optical biometric parameter, and/or parameters relating to the subject's personal information.

According to various embodiments, a circuit may include analog circuits or components, digital circuits or components, or hybrid circuits or components. Any other kind of implementation of the respective functions which will be described in more detail below may also be understood as a "circuit" in accordance with an alternative embodiment. A digital circuit may be understood as any kind of a logic implementing entity, which may be special purpose circuitry or a processor executing software stored in a memory, firmware, or any combination thereof. Thus, in various embodiments, a "circuit" may be a digital circuit, e.g. a hard-wired logic circuit or a programmable logic circuit such as a programmable processor, e.g. a microprocessor (e.g. a Complex Instruction Set Computer (CISC) processor or a Reduced Instruction Set Computer (RISC) processor). A "circuit" may also include a processor executing software, e.g. any kind of computer program, e.g. a computer program using a virtual machine code such as e.g. Java.

System 700 further includes means for determining the subject's risk of the onset or progression of myopia over a timeframe 720, based on the determined value of the at least one parameter. Said means for determining the subject's risk of the onset or progression of myopia over a timeframe 720 may include at least one circuit configured to perform the steps of methods 400, 500A, 500B and 600. According to various embodiments, the means for determining the subject's risk of the onset or progression of myopia over a timeframe 720, e.g. circuit or microprocessor, may include a computer program or instructions. In one example, the computer program or instructions may be executed to perform the steps of methods 400, 500A, 500B, to determine if the subject's risk of myopia onset or progression over a timeframe is of the low-risk, moderate-risk, or high-risk category. In another example, the instructions may be executed to perform the steps of method 600, to calculate the value of the risk ratio which provides a probability indicative of the subject's risk of myopia onset or progression over the timeframe. According to various embodiments, the computer program or instructions may be implemented in the at least one predictive model based on the machine learning algorithm.

According to various embodiments, the means for determining the value of the at least one parameter associated with vision condition of the subject 710 and the means for determining the subject's risk of the onset or progression 720 may include a circuit, e.g. microprocessor. In an embodiment, the means for determining the value of the at least one parameter associated with vision condition of the subject 710 and the means for determining the subject's risk of the onset or progression 720 may be implemented in the same circuit, e.g. microprocessor. For example, the means for determining the subject's risk of the onset or progression 720, for instance, the instructions and computer program to execute the steps of methods 400, 500A, 500B and 600 may be implemented in the same circuit as the means for determining the value of the at least one parameter associated with vision condition of the subject 710 to execute step 110 of method 100 and 300. As a further example, the means for determining the subject's risk of the onset or progression 720 may be implemented in the ophthalmic testing device used to determine the value of the of the at least one parameter associated with vision condition of the subject 710.

Advantageously, including the means for determining the value of the at least one parameter associated with vision condition of the subject 710 and the means for determining the subject's risk of the onset or progression over the timeframe 720 in the same circuit, provides a direct and simple way of determining said subject's risk. For example, the operator operating the circuit does not need to perform any additional steps to determine said risk.

In some embodiments, the means for determining the value of the at least one parameter associated with vision condition of the subject 710 and the means for determining the subject's risk of the onset or progression 720 may each include a circuit. In other words, the means for determining the value of the at least one parameter associated with vision condition of the subject 710 and the means for determining the subject's risk of the onset or progression 720 may be implemented in separate circuits. For example, the means for determining the value of the at least one parameter associated with vision condition of the subject 710 may be obtained and calculated on an ophthalmic testing device, and the means for determining the subject's risk of the onset or progression 720 may be implemented on another microprocessor. In this embodiment, the determined value of the at least one parameter associated with vision condition of the subject, obtained at step 110, may be transmitted according to a pre-defined wireless communication protocol to the circuit to determine the subject's risk of the onset or progression of myopia over the timeframe 720. Examples of the pre-defined wireless communication protocols include: global system for mobile communication (GSM), enhanced data GSM environment (EDGE), wideband code division multiple access (WCDMA), code division multiple access (CDMA), time division multiple access (TDMA), wireless fidelity (Wi-Fi), voice over Internet protocol (VoIP), worldwide interoperability for microwave access (Wi-MAX), Wi-Fi direct (WFD), an ultra-wideband (UWB), infrared data association (IrDA), Bluetooth, ZigBee, SigFox, LPWan, LoRaWan, GPRS, 3G, 4G, LTE, and 5G communication systems. Accordingly, each of the circuits may include the necessary hardware required to support the wireless transmission. Alternatively, it is envisioned that the determined value of the at least one parameter associated with vision condition of the subject may be transmitted via wired means.

According to various embodiments, a computer program product may include instructions to cause the system 700 to execute the steps of methods 100, 300, 400, 500A, 500B and 600. The steps of methods 100, 300, 400, 500A, 500B and 600 may be used to determine the subject's risk of onset or progression of myopia over the timeframe.

According to various embodiments, the methods and system of the present invention may further include providing a recommendation for mitigating the risk for the non-myopic or myopic subject. For example, mitigation options may be recommended if it is determined that the subject has a moderate-risk or high-risk of myopia onset, or a fast or high rate of progression of myopia over a timeframe. The mitigation options may include but are not limited to: lens(es) configured to control myopia progression; atropine eye drops; orthokeratology; multifocal soft contact lenses; spectacle lenses designed to slow the progression of myopia; a change in the subject's lifestyle, activity or behavior, e.g. increased duration spent outdoors in natural light per day, reduction in the amount of time spent on work involving near vision, change in subject's nutrition, or any combination thereof; the recommendation to perform further tests on the subject, e.g. further optometric exams, genetic testing, imaging one or both eyes of the individual, screening for ocular disorders or conditions, or any combinations thereof.

Advantageously, the present invention uses the value of the sensitivity parameter SRx of the subject, as a novel factor for determining a subject's risk of an onset or progression of myopia over a timeframe. Specifically, the lack of sensitivity to refraction, i.e. the value of the sensitivity parameter SRx, and/or the value of the near and far refraction sensitivity parameters, provide a direct indication of a level of image degradation that a subject can perceive, and which is directly related to myopia onset or progression. As a result, the value of the sensitivity parameter SRx, and additionally, the overlap degree of the subject's far and near comfort zones (based on the subject's far refraction sensitivity and near refraction sensitivity) provide additional factors which improves the predictive power of existing or new myopia prediction models. Identification of the risk of myopia onset or progression for the subject will allow early intervention to mitigate said risk.

EXAMPLES

The system 700 and methods 400, 500A, 500B, 600, herein disclosed are further illustrated in the following examples, which are provided by way of illustration and are not intended to be limiting the scope of the present disclosure.

The reference values or reference value range in the tables shown in FIGS. 8A to 9C provide exemplary preferred threshold values for the limits of sensitivity in each category. For example, the limit in sensitivity of the sensitivity parameter SRx may range from 0 to 0.15 D for the low-risk category, from 0.15 to 0.3 D for the medium-risk category, and > 0.3 D for the high-risk category. Alternatively, the limits in the sensitivity of the sensitivity parameter SRx may range from 0.1 to 0.2 D for the low-risk category, from 0.2 to 0.4 D for the medium-risk category, and > 0.4 D for the high-risk category. As indicated above, the reference values or reference value range for the respective parameters, e.g. parameter relating to the subject's genetic history, dioptric optical parameter, sensitivity parameter SRx, may be established on the basis of a database comprising information of the risk of myopia onset or progression over the timeframe, for individuals within a population sample.

***Example 1. Pre-myopic subject and use of the sensitivity parameter SRx***

FIG. 8A and 8B show tables of an example of a use condition of methods 400, 500A and 500B, to determine the subject's risk of myopia onset or progression over a timeframe. In this example, the subject may be an eight-year-old female. Referring to FIG. 8A, it may be determined that the dioptric optical parameter of the subject is plano (indicated by circles), e.g. subject is non-myopic, obtained based on an eye examination. Other parameters may include the subject's genetic history and the parameter relating to the subject's lifestyle, activity or behavior. In this example, the subject indicates that she has one myopic parent (indicated by circles), and the subject's parameter relating to lifestyle, activity or behavior, i.e. time spent outdoors per day, is greater than 2 hours (indicated by circles). Said parameters may be entered into a myopia onset predictive model as shown in FIG. 8A.

As may be seen from FIG. 8A, the subject has one factor, e.g. dioptric optical parameter, that falls in the high-risk category, one factor, e.g. genetic history that falls in the moderate-risk category, and one factor, e.g. lifestyle, activity or behavior that falls in the low-risk category. The reference value or reference value range within each of the low-, moderate- and high-risk categories may be established on the basis of a database comprising information of the risk of myopia onset or progression over the timeframe, for individuals within a population sample. Since each risk category, e.g. plurality of predetermined risk categories includes one factor each, it may be difficult to determine the subject's risk of the onset or progression of myopia over the timeframe.

The subject's sensitivity parameter SRx may then be further determined. In this example, the subject's sensitivity parameter SRx may be determined to have a value of 0.37 D. Referring to FIG. 8B, it may be seen that the subject's sensitivity parameter SRx of 0.37 D falls within the high-risk category, and accordingly the high-risk category now includes two factors, e.g. dioptric optical parameter and the sensitivity parameter SRx, and the moderate- and low-risk categories each include one parameter. It may then be determined that the subject has a high-risk of developing myopia within a timeframe and appropriate options to mitigate said risk may be recommended. For example, low dosage atropine eye drops may be prescribed, or the subject may be recommended to increase the duration of time spent outdoors per day.

Alternatively, the subject's risk of myopia onset or progression over a timeframe may be determined using method 500B which includes assigning a weight to each of the low-, moderate- and high-risk categories. For example, the low-risk category may include a weight of "*0*", the moderate-risk category a weight of "*1*" and the high-risk category a weight of "2" (step 520 of method 500). By performing steps 522 and 526, a final score of 5, e.g. *final score* = (0)(1) + (1)(1) + (2)(2), may be calculated. Step 528 may include comparing the final score with at least one first predetermined threshold value. For example, the final score may be compared against first predetermined threshold values for each of the low-risk, moderate-risk, and high-risk categories, and at step 530, the subject's risk of the onset or progression of myopia over the timeframe may be determined.

Alternatively, the subject's risk of myopia onset or progression over a timeframe may be determined by calculating a value of a risk ratio, e.g. percentage according to method 600, which is carried out using the at least one predictive model based on a machine learning algorithm.

Therefore, as shown in FIG. 8B, it may be seen that advantageously, the use of the sensitivity parameter SRx as an additional parameter within an existing myopia predictive model improves the predictive power of the model.

***Example 2. Myopic subject and use of the sensitivity parameter SRx and fifth parameter***

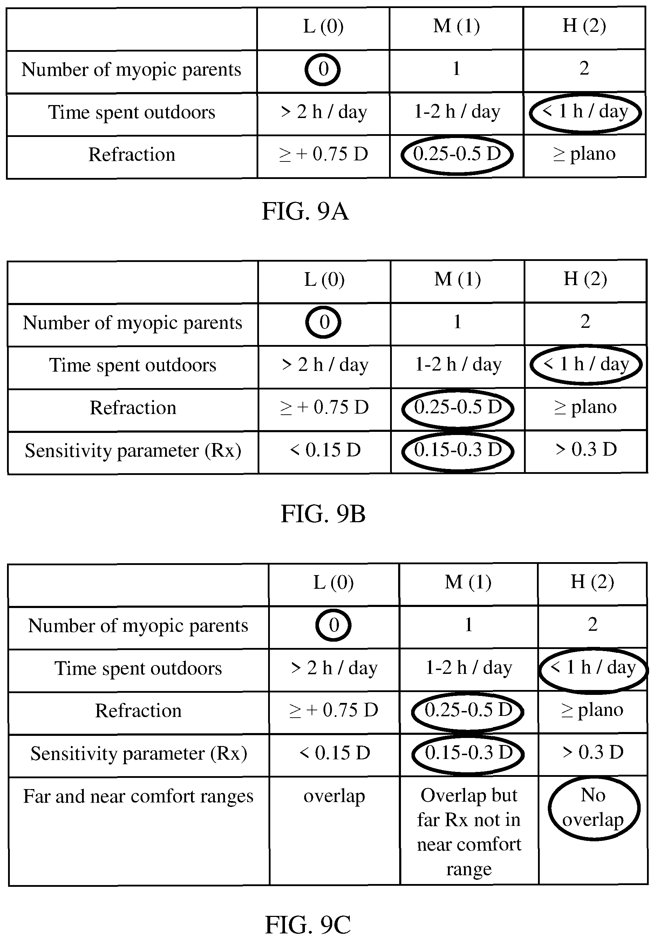

FIG. 9A, 9B and 9C show tables of an example of a use condition of according to methods 300, 400, 500A and 500B, to determine the subject's risk of myopia onset or progression over a timeframe. In this example, the subject may be a six-year-old male. Referring to FIG. 9A, it may be determined that the dioptric optical parameter of the subject ranges between 0.25-0.50 D (indicated by circles), e.g. subject has low hyperopia, obtained based on an eye examination. Other parameters may include the subject's genetic history and the parameter relating to the subject's lifestyle, activity or behavior. In this example, the subject indicates that he does not have any myopic parents (indicated by circles), and that the subject's time spent outdoors per day is less than 1 hour (indicated by circles). Said parameters may be entered into a myopia onset predictive model as shown in FIG. 9A.

As may be seen from FIG. 9A, the subject has one factor, e.g. lifestyle, activity or behavior, that falls in the high-risk category, one factor, e.g. dioptric optical parameter that falls in the moderate-risk category, and one factor, e.g. genetic history, that falls in the low-risk category. Since each risk category of the plurality of predetermined risk categories includes one factor each, it may be difficult to determine the risk of the subject's onset or progression of myopia over the timeframe.

The subject's sensitivity parameter SRx may then be further determined. In this example, the subject's sensitivity parameter SRx may be determined to have a value of ranging between 0.15-0.3 D, which corresponds to the sensitivity parameter SRx of the moderate-risk category. As shown in FIG. 9B, it may be determined that the subject has a moderate-risk of myopia progression within the timeframe. However, there is still one factor which falls in the high-risk category.

Thus, the subject's first and third parameters indicating a near and far refraction of the subject may be obtained (step 310), and in this example may be measured as +0.5 D and +2.0 D, respectively. At step 320, the second and fourth parameters indicating a near and far refraction sensitivity of the subject may be determined, and in this example may be calculated as being equal to 0.5 D and 0.2 D, respectively. Step 330 includes determining the far and near comfort ranges according to Equations 2 and 3, using subject specific coefficients *K_near* and *K_far* of 2. For example, the far and near comfort range may be determined as:

$$Far\ comfort\ range = 0.5 \pm (2 \times 0.2) = [+0.9 \text{ to } +0.1]\,\mathrm{D}$$

$$Near\ comfort\ range = 2.0 \pm (2 \times 0.5) = [+3.0 \text{ to } +1.0]\,\mathrm{D}$$

At step 340, the overlap degree between the far and near comfort ranges may be determined, and at step 350, a value may be assigned to the fifth parameter which indicates the overlap degree of the far and near comfort ranges. In this example, it may be seen that there is no overlap between the far and comfort ranges, e.g. value assigned to fifth parameter may be *"no overlap",* which falls within the high-risk category, as shown in FIG. 9C.

Based on steps 510 and 512 of method 500A, it may thus be determined that the subject has a high-risk of fast myopia progression within the timeframe, and appropriate options to mitigate said risk may be recommended. In other words, with the addition parameter indicating the overlap degree between the subject's near and far comfort ranges, which is determined based on the subject's near and far refraction sensitivity, the predictive power of the myopia model may be improved.

Alternatively, the subject's risk of myopia onset or progression over a timeframe may be determined using method 500B which includes step 520 of assigning a weight to each of the low-, moderate- and high-risk categories; step 522 of determining, for each risk category of the plurality of predetermined risk categories, the number of parameters among the at least one parameter, to which the respective risk category has been assigned; step 524 of calculating, for each risk category of the plurality of predetermined risk categories, a score by multiplying the weight assigned to the respective risk category with the number of parameters, to which said risk category has been assigned; step 526 of calculating, a final score by summing the scores calculated for the plurality of predetermined risk categories; step 528 of comparing the final score with at least one first predetermined threshold value; and step 530 of determining, the subject's risk of the onset or progression of myopia over the timeframe based on the final score.

Alternatively, the subject's risk of myopia onset or progression over a timeframe may be determined by calculating a value of a risk ratio, e.g. percentage according to method 600, which is carried out using predictive models based on a machine learning algorithm.

Therefore, as shown in FIG. 9C, it may be seen that advantageously, the use of the sensitivity parameter SRx and the fifth parameter indicating the overlap degree between the subject's near and far comfort ranges as additional parameters in a myopia predictive model improves the predictive power of the model. Alternatively, the fifth parameter may be the sole or major determining factor in the myopia predictive model. For example, the subject may be at a high-risk of fast myopia progression within the timeframe if the fifth parameter falls within the high-risk category, even though the values of the parameters in the other categories, e.g. genetic history, the parameter relating to the subject's lifestyle, activity or behavior, the dioptric optical parameter and/or the sensitivity parameter SRx may fall within the low-risk category.

### Example 3. Myopia progression

In this example, the subject may be an eleven-year-old myopic male. The subject has indicated that his prescription, e.g. dioptric optical parameters remains the same as that in the previous year, e.g. stable refraction, but complains of intermittent blurred vision after engaging in near work for long durations.

In this example, the optometrist (or the clinician) only has a few parameters, namely, personal information about the subject, e.g. age and/or gender, and the rate of myopia progression in the previous year (which is a weak indicator). However, the optometrist may have access to the subject's sensitivity parameter SRx, which for the subject was measured to be 0.52 D, e.g. based on a previous sensitivity parameter SRx, which places the subject in the high-risk category, indicating that the subject, despite stable refraction, will likely to be prone to fast myopia progression. The use of the subject's sensitivity parameter SRx may thus provide an additional powerful parameter, in particular, when the optometrist has limited information about the subject, to improve the predictive power of the existing myopia predictive model.

While the disclosure has been particularly shown and described with reference to specific embodiments, it should be understood by those skilled in the art that various changes in form and detail may be made therein without departing from the spirit and scope of the invention as defined by the appended claims. The scope of the invention is thus indicated by the appended claims and all changes which come within the meaning and range of equivalency of the claims are therefore intended to be embraced.

### Claims

1. A method (100) for determining a risk of an onset or progression of myopia over a timeframe, the method (100) comprising:

   - determining a value of at least one parameter associated with vision condition of a subject (110), said at least one parameter comprising a sensitivity parameter (SRx) of said subject, said sensitivity parameter (SRx) being relative to the sensitivity of said subject to a variation of at least one dioptric optical feature of at least one ophthalmic lens placed in front of at least one eye of said subject; and
   - determining the subject's risk of the onset or progression of myopia over the timeframe, based on the determined value of the at least one parameter (120).

2. The method (100) of claim 1, wherein the at least one parameter associated with vision condition of the subject further comprises: a dioptric optical parameter, a parameter relating to the subject's lifestyle, activity or behavior, a parameter relating to the subject's genetic history, an optical biometric parameter, and/or a parameter relating to personal information about the subject.

3. The method (100) of any one of claims 1 to 2, wherein determining the subject's risk of the onset or progression of myopia over the timeframe, based on the determined value of the at least one parameter (120), further comprises:

   - assigning to each of the at least one parameter, a respective risk category selected from a plurality of predetermined risk categories, based on the determined value of the respective parameter (420); and
   - determining the subject's risk of the onset or progression of myopia over the timeframe, based on the respective risk category assigned to each of the at least one parameter (430).

4. The method (100) of claim 3, wherein assigning the respective risk category to each of the at least one parameter, based on the determined value of the respective parameter (420) comprises:

   - determining if the determined value of the respective parameter matches a corresponding reference value or lies within a corresponding reference value range, for the respective parameter associated with the respective risk category (410).

5. The method (100) of claim 4, wherein the reference value or reference value range for the respective parameter, is established on the basis of a database comprising information of the risk of myopia onset or progression over the timeframe, for individuals within a population sample.

6. The method (100) of any one of claims 3 to 5, wherein determining the subject's risk of the onset or progression of myopia over the timeframe, based on the respective risk category assigned to each of the at least one parameter (420) comprises:

- determining for each risk category of the plurality of predetermined risk categories, the number of parameters among the at least one parameter, to which the respective risk category has been assigned (510);
- determining as the subj ect's risk of the onset or progression of myopia over the timeframe, that risk category of the plurality of predetermined risk categories, that has been assigned to a highest number of parameters (512).

7. The method (100) of any one of claims 3 to 5, wherein determining the subject's risk of the onset or progression of myopia over the timeframe, based on the respective risk category assigned to each of the at least one parameter (420) comprises:

- assigning a weight to each one of the plurality of predetermined risk categories (520);
- determining for each risk category of the plurality of predetermined risk categories, the number of parameters among the at least one parameter, to which the respective risk category has been assigned (522);
- calculating for each risk category of the plurality of predetermined risk categories, a score by multiplying the weight assigned to the respective risk category with the number of parameters, to which said risk category has been assigned (524),
- calculating a final score by summing the scores calculated for the plurality of predetermined risk categories (526), and
- determining the subject's risk of the onset or progression of myopia over the timeframe based on the final score (530).

8. The method (100) of claim 7, wherein determining the subject's risk of the onset or progression of myopia over the timeframe based on the final score (530) comprises, comparing the final score with at least one first predetermined threshold value (528).

9. The method (100) of any one of claims 1 to 8, wherein determining the subject's risk of the onset or progression of myopia over the timeframe, based on the determined value of the at least one parameter (120), is established based on at least one predictive model based on a machine learning algorithm, which provides a relationship between the risk of myopia onset or progression and the at least one parameter.

10. The method (100) of claim 9, wherein determining the subject's risk of the onset or progression of myopia over the timeframe, based on the determined value of the at least one parameter (120) further comprises:

- entering the determined value of the at least one parameter into the at least one predictive model based on the machine learning algorithm (610);
- calculating a value of a risk ratio, using the at least one predictive model based on the machine learning algorithm (620); and
- determining the subject's risk of the onset or progression of myopia over the timeframe based on the calculated value of the risk ratio (630);

wherein the calculated value of the risk ratio (630) provides a probability indicative of the subject's risk of the onset or progression of myopia over the timeframe, and is carried out using the at least one predictive model based on the machine learning algorithm.

11. The method (100) of claim 10, wherein determining the subject's risk of the onset or progression of myopia over the timeframe based on the risk ratio (630) comprises, comparing the calculated value of the risk ratio with at least one second predetermined threshold value.

12. The method (100) of any one of claims 1 to 11, wherein the at least one parameter associated with vision condition of said subject, comprises:

- a first parameter indicating a near refraction of said subject (310);
- a second parameter indicating a near refraction sensitivity of said subject (320);
- a third parameter indicating a far refraction of said subject (310);
- a fourth parameter indicating a far refraction sensitivity of said subject (320); and

- a fifth parameter indicating an overlap degree of a far comfort range and a near comfort range of said subject (350);

wherein the determining the value of the fifth parameter (350) comprises:

- determining the far comfort range and the near comfort range based on the determined values of the first to fourth parameters (330),
- determining the overlap degree of the far and near comfort ranges (340), and
- assigning a value to the fifth parameter based on the determined overlap degree of the far and near comfort ranges (350).

13. A system (700) for determining a risk of an onset or progression of myopia over a timeframe, the system comprising:

- means for determining a value of at least one parameter associated with vision condition of a subject (710), said at least one parameter comprising a sensitivity parameter (SRx) of said subject, said sensitivity parameter (SRx) being relative to the sensitivity of said subject to a variation of at least one dioptric optical feature of at least one ophthalmic lens placed in front of at least one eye of said subject; and
- means for determining the subject's risk of the onset or progression of myopia over the timeframe, based on the determined value of the at least one parameter (720).

14. The system (700) of claim 13, wherein at least one of the means for determining the value of the at least one parameter, and the means for determining the subject's risk of the onset or progression of myopia, comprises a circuit.

15. A computer program product, comprising instructions to cause the system (700) of claims 13 or 14 to execute the steps of the method (100, 300, 400, 500A, 500B, 600) of any one of claims 1 to 12.

100

Determine a value of at least one parameter associated with subject's vision condition ⌐110

↓

Determine subject's risk of onset or progression of myopia over a timeframe ⌐120

FIG. 1

200

Subject certitude probability function

◇ Measured

—— Estimated

+100%

100% sure choice 2

+50%

SRx

0%

210

-50%

230

100% sure choice 1

±Sensitivity ——220

Rx -100%

-0.4        -0.2        0        0.2        0.4

Estimated parameter value (δ)

FIG. 2

<u>300</u>

```
┌─────────────────────────────────┐
│                                 │╮ 310
│  Determine first and third      │
│  parameters                     │
└─────────────────────────────────┘
             │
             ▼
┌─────────────────────────────────┐
│                                 │╮ 320
│  Determine second and fourth    │
│  parameters                     │
└─────────────────────────────────┘
             │
             ▼
┌─────────────────────────────────┐
│                                 │╮ 330
│  Determine far and near         │
│  comfort range                  │
└─────────────────────────────────┘
             │
             ▼
┌─────────────────────────────────┐
│  Determine overlap degree of    │╮ 340
│  far and near comfort ranges    │
└─────────────────────────────────┘
             │
             ▼
┌─────────────────────────────────┐
│  Assign value to fifth          │╮ 350
│  parameter indicating overlap   │
│  degree of far and near comfort │
│  ranges                         │
└─────────────────────────────────┘
```

FIG. 3

400

```
┌─────────────────────────────────────┐
│ Determine if determined value of     │
│ parameter matches reference value    │──── 410
│ or lies within reference value range │
│ associated with respective risk      │
│ category                             │
└─────────────────────────────────────┘
                  │
                  ▼
┌─────────────────────────────────────┐
│                                      │
│ Assign respective risk category to   │──── 420
│ parameter                            │
│                                      │
└─────────────────────────────────────┘
                  │
                  ▼
┌─────────────────────────────────────┐
│ Determine subject's risk of onset    │
│ or progression of myopia over a      │──── 430
│ timeframe based on respective        │
│ assigned risk category               │
└─────────────────────────────────────┘
```

FIG. 4

500A

```
┌─────────────────────────────────────┐
│ Determine number of parameters       │
│ within each risk category            │──── 510
│                                      │
└─────────────────────────────────────┘
                  │
                  ▼
┌─────────────────────────────────────┐
│ Determine subject's risk of onset    │
│ or progression of myopia over a      │
│ timeframe as risk category with      │──── 512
│ highest number of assigned           │
│ parameters                           │
└─────────────────────────────────────┘
```

FIG. 5A

<u>500B</u>

Assign weight to each risk category — 520

Determine number of parameters within each risk category — 522

Calculate score for each risk category — 524

Calculate final score — 526

Compare final score to at least one first pre-determined threshold value — 528

Determine the subject's risk of onset or progression of myopia over the timeframe based on comparison — 530

FIG. 5B

<u>600</u>

```
┌─────────────────────────────────────┐
│  Enter determined value of parameter │──── 610
│         into predictive model        │
└─────────────────────────────────────┘
                   │
                   ▼
┌─────────────────────────────────────┐
│      Calculate value of risk ratio   │──── 620
└─────────────────────────────────────┘
                   │
                   ▼
┌─────────────────────────────────────┐
│     Determine subject's risk of onset│
│   or progression of myopia over a    │──── 630
│      timeframe based on value of     │
│              risk ratio              │
└─────────────────────────────────────┘
                   │
                   ▼
┌─────────────────────────────────────┐
│   Compare value of risk ratio to     │──── 640
│   second pre-determined threshold    │
│                value                 │
└─────────────────────────────────────┘
```

FIG. 6

FIG. 7

| Parameter | L (0) | M (1) | H (2) |
|---|---|---|---|
| Genetic history | 0 | ①) | 2 |
| Lifestyle, activity, behavior | (> 2 h / day) | 1-2 h / day | < 1 h / day |
| Dioptric optical | ≥ + 0.75 D | 0.25-0.5 D | (≥ plano) |

FIG. 8A

| | L (0) | M (1) | H (2) |
|---|---|---|---|
| Genetic history | 0 | ①) | 2 |
| Lifestyle, activity, behavior | (> 2 h / day) | 1-2 h / day | < 1 h / day |
| Dioptric optical | ≥ + 0.75 D | 0.25-0.5 D | (≥ plano) |
| Sensitivity parameter (Rx) | < 0.15 D | 0.15-0.3 D | (> 0.3 D) |

FIG. 8B

| | L (0) | M (1) | H (2) |
|---|---|---|---|
| Number of myopic parents | (0) | 1 | 2 |
| Time spent outdoors | > 2 h / day | 1-2 h / day | (< 1 h / day) |
| Refraction | ≥ + 0.75 D | (0.25-0.5 D) | ≥ plano |

FIG. 9A

| | L (0) | M (1) | H (2) |
|---|---|---|---|
| Number of myopic parents | (0) | 1 | 2 |
| Time spent outdoors | > 2 h / day | 1-2 h / day | (< 1 h / day) |
| Refraction | ≥ + 0.75 D | (0.25-0.5 D) | ≥ plano |
| Sensitivity parameter (Rx) | < 0.15 D | (0.15-0.3 D) | > 0.3 D |

FIG. 9B

| | L (0) | M (1) | H (2) |
|---|---|---|---|
| Number of myopic parents | (0) | 1 | 2 |
| Time spent outdoors | > 2 h / day | 1-2 h / day | (< 1 h / day) |
| Refraction | ≥ + 0.75 D | (0.25-0.5 D) | ≥ plano |
| Sensitivity parameter (Rx) | < 0.15 D | (0.15-0.3 D) | > 0.3 D |
| Far and near comfort ranges | overlap | Overlap but far Rx not in near comfort range | (No overlap) |

FIG. 9C

Europäisches
Patentamt

European
Patent Office

Office européen
des brevets

# EUROPEAN SEARCH REPORT

**Application Number**

**EP 21 30 6550**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/083382 A1 (AI TECH INC; LI ZHIHUAN [CN]) 30 April 2020 (2020-04-30) * claims 1, 13, 15 * ----- | 1-15 | INV. G16H50/30 |
| A,D | US 2021/311325 A1 (MARIN GILDAS [FR] ET AL) 7 October 2021 (2021-10-07) * claim 1; figure 1 * ----- | 1-15 | |

**TECHNICAL FIELDS SEARCHED (IPC)**

G16H

The present search report has been drawn up for all claims

| Place of search | Date of completion of the search | Examiner |
|---|---|---|
| Munich | 4 April 2022 | Beligny, Samuel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.......................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 30 6550

This annex lists the patent family members relating to the patent documents cited in the above-mentioned European search report.
The members are as contained in the European Patent Office EDP file on
The European Patent Office is in no way liable for these particulars which are merely given for the purpose of information.

04-04-2022

| Patent document cited in search report | | Publication date | Patent family member(s) | | Publication date |
|---|---|---|---|---|---|
| WO 2020083382 | A1 | 30-04-2020 | CN | 113196317 A | 30-07-2021 |
| | | | EP | 3871170 A1 | 01-09-2021 |
| | | | US | 2021375460 A1 | 02-12-2021 |
| | | | WO | 2020083382 A1 | 30-04-2020 |
| US 2021311325 | A1 | 07-10-2021 | BR | 112020026207 A2 | 23-03-2021 |
| | | | CA | 3107059 A1 | 23-01-2020 |
| | | | CN | 112449687 A | 05-03-2021 |
| | | | EP | 3598211 A1 | 22-01-2020 |
| | | | EP | 3824341 A1 | 26-05-2021 |
| | | | JP | 2021531506 A | 18-11-2021 |
| | | | KR | 20210031433 A | 19-03-2021 |
| | | | US | 2021311325 A1 | 07-10-2021 |
| | | | WO | 2020016398 A1 | 23-01-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020016398 A
- WO 2018015381 A
- WO 2020126513 A

**Non-patent literature cited in the description**

- **ROSENFIELD M et al.** *Optom. Vis. Sci.,* 1999, vol. 76 (5), 303-307
- **DROBE B et al.** *Ophthal. Physiol. Opt.,* 1995, vol. 15, 375-378